# FASCICULE DE BREVET EUROPEEN

(11) **EP 0 395 455 B1**
(45) Date de publication et mention de la délivrance du brevet: **04.08.1993**
(21) Numéro de dépôt: 90400598.0
(22) Date de dépôt: 05.03.1990
(51) Int. Cl.: C07C 51/353, C07C 51/347, C07C 53/128, C07C 61/08, C07C 61/13, C07C 61/135

(54) **Procédé de fabrication d'acides carboxyliques par réaction d'alcanes et de formiates**
Verfahren zur Herstellung von Carbonsäuren durch die Umsetzung von Alkanen und Formiaten
Process for preparing carboxylic acids by the reaction of alkanes and formiates

(30) Priorité: 18.04.1989 FR 8905153
(43) Date de publication de la demande: 31.10.1990
(73) Titulaire: ELF ATOCHEM S.A., 92800 Puteaux (FR)
(72) Inventeur: Lazzari, Véronique, F-13005 Marseille (FR); Jalal, Rachid, F-13014 Marseille (FR); Gallo, Roger, F-13320 Bouc Bel Air (FR)
(74) Mandataire: Rochet, Michel

(56) Documents cités:
- CH-A- 396 866
- FR-A- 1 251 646

## Description

La présente invention porte sur un procédé de fabrication d'acides carboxyliques dans lesquels l'atome de carbone en alpha du groupe carboxyle est un atome de carbone tertiaire, ce procédé consistant à faire réagir les alcanes correspondants sur les formiates.

La synthèse de Koch des acides carboxyliques ramifiés s'effectue par réaction d'oléfines avec l'oxyde de carbone et l'eau, catalysée par des acides minéraux, tels que l'acide sulfurique ou l'acide phosphorique, à température et pression modérées : 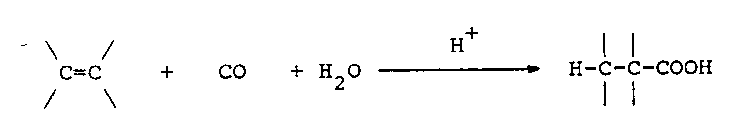
où :
〉C=C〈 représente généralement l'isobutène ou une oléfine en C₅-C₁₀.

La réaction de Koch et Haaf est une variante à pression atmosphérique de la réaction de Koch, où l'oxyde de carbone est apporté par la décomposition de l'acide formique et où le dérivé à carbonyler peut être une oléfine ou un dérivé fonctionnel équivalent (F. Falbe, «New syntheses with carbon monoxide», Springer, Weinheim, 1980) : 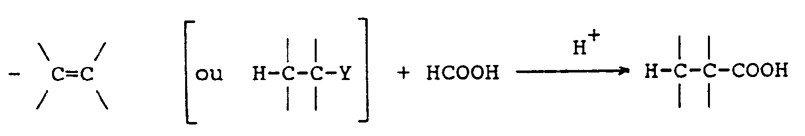
- 〉C=C〈 est tel que défini ci-dessus ; et
- Y représente notamment un reste hydroxyle, halogéno ou alcoxyle.

On connaît également une méthode de synthèse directe des esters carboxyliques par réaction d'une oléfine et d'un formiate d'alkyle en présence d'un acide minéral comme l'acide sulfurique (EP-A-0 219 948 ; EP-A-0 092 350 ; EP-A-0 106 656) : 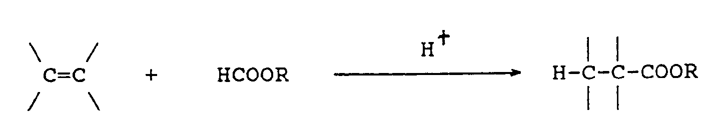
où :
- 〉C=C〈 est tel que défini ci-dessus
- R représente, de préférence, un groupe méthyle ou encore un groupe alkyle primaire en C₂-C₁₀.

Il est également possible, d'après les travaux de Haaf et Koch, de préparer, à pression atmosphérique, des acides carboxyliques à partir d'alcanes, en utilisant l'acide formique comme source d'oxyde de carbone et un dérivé fonctionnel RY, qui sert à activer l'alcane à carbonyler : 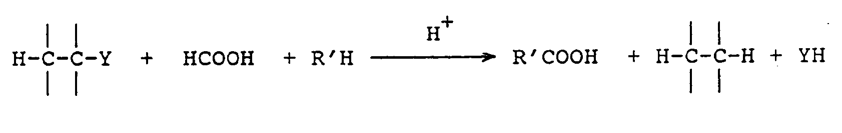
où :
- R' représente un isoalcane ; et
- Y est tel que défini ci-dessus.

La Société déposante a maintenant découvert que l'on peut obtenir des acides carboxyliques du type indiqué ci-dessus par réaction directe d'un alcane avec un formiate, sans qu'il soit nécessaire d'ajouter de l'acide formique au milieu réactionnel.

L'intérêt du procédé selon l'invention est qu'il permet d'obtenir des acides carboxyliques du type précité par carbonylation à température et pression ambiantes. Par ailleurs, cette réaction peut être effectuée à l'aide d'alcanes ou de coupes d'alcanes, dont la transformation chimique est difficile. Enfin, un intérêt supplémentaire du procédé est dû au fait qu'il permet d'utiliser des formiates (pouvant être obtenus industriellement par piégeage d'oxyde de carbone dans les effluents gazeux de certains procédés industriels) qui servent de transporteur liquide d'oxyde de carbone et d'activateur d'alcane.

La présente invention a donc d'abord pour objet un procédé de fabrication d'un acide carboxylique représenté par la formule (I) ci-après :

R¹ - COOH (I)

dans laquelle R¹ représente un reste alkyle ramifié tertiaire ou bien un reste cycloalkyle à un ou plusieurs cycles accolés ou non, et éventuellement substitué par au moins un reste alkyle, l'atome de carbone en alpha du groupe carbonyle étant un atome de carbone tertiaire,
caractérisé par le fait que l'on fait réagir, en présence d'un acide comme catalyseur, un alcane ramifié ou cyclique de formule (II) :

R¹H (II)

dans laquelle R¹ est un reste alkyle ramifié ou bien un reste cycloalkyle à un ou plusieurs cycles accolés ou non, et éventuellement substitué par au moins un reste alkyle, l'atome de carbone en alpha de l'atome d'hydrogène étant un atome de carbone tertiaire ou un atome de carbone secondaire capable de se réarranger en cours de réaction en atome tertiaire,
avec un formiate de formule (III) : 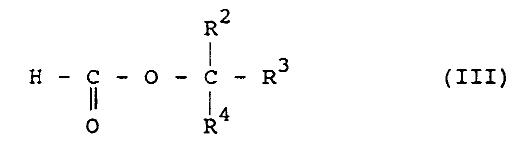
dans laquelle R², R³ et R⁴ représentent chacun indépendamment un atome d'hydrogène ou un reste alkyle, à la triple condition que R², R³ et R⁴ ne représentent pas simultanément un atome d'hydrogène, que le composé (III) conduise dans le milieu réactionnel, directement, ou à la suite d'un réarrangement, à un cation R²R³R⁴C⁺ stable, et que l'alcane R²R³R⁴CH qui se forme puisse s'éliminer aisément du milieu réactionnel.

Le schéma réactionnel est donc le suivant :

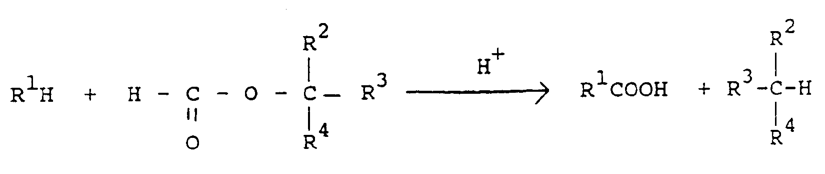

Dans cette nouvelle réaction, le formiate de formule (III) se décompose, en présence d'un acide, pour donner de l'oxyde de carbone et un cation R²R³R⁴C⁺.

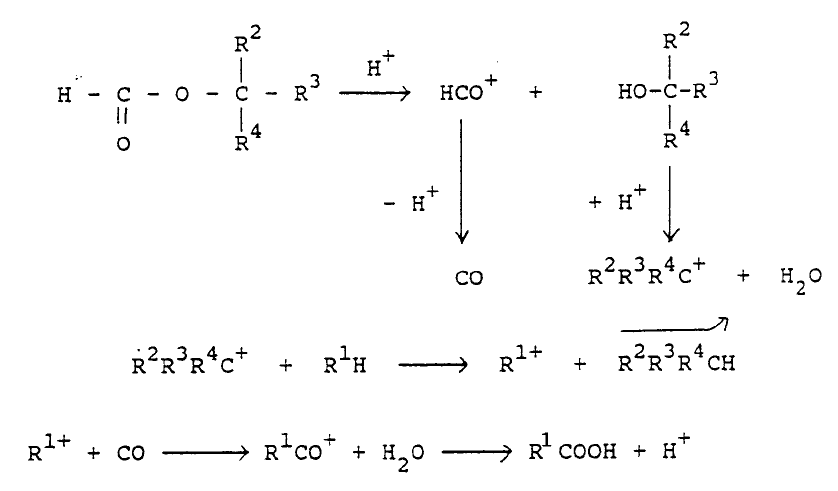

Par rapport aux réactions de carbonylation de Haaf et Koch effectuées à partir d'alcanes, il n'est plus nécessaire de faire intervenir un dérivé fonctionnel 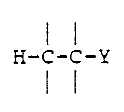
(RY) pour donner un cation tertiaire stable nécessaire au transfert d'hydrure, ni de l'acide formique pour fournir du monoxyde de carbone, du fait que le formiate de formule (III) se décompose en donnant in situ ces deux réactifs. De plus, par rapport aux réactions décrites par Haaf, il n'y a pas de dégagement d'eau (ce qui est le cas lorsque RY = ROH), qui dilue le milieu réactionnel et diminue l'activité du mélange, ou d'acide HX (ce qui est le cas lorsque RY = halogénure d'alkyle RX), qui possède un effet corrosif dans le milieu. On prépare notamment des acides carboxyliques de formule (Ia) : 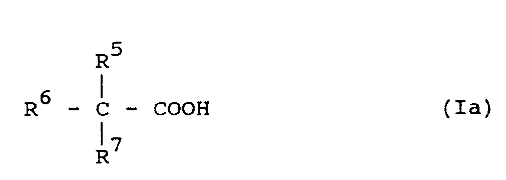
dans laquelle :
- R⁵, R⁶ et R⁷ représentent chacun indépendamment un reste alkyle en C₁-C₁₀ ; ou bien
- deux radicaux des R⁵ à R⁷ sont réunis pour former un enchaînement -(CH₂)ₙ- , n étant un nombre entier allant de 4 à 11, et le troisième représente un reste alkyle en C₁-C₇ ; ou bien
- R⁵, R⁶ et R⁷ sont combinés entre eux et avec l'atome de carbone qui les porte pour former un reste cycloalkyle à un, deux ou trois cycles.

Comme alcane de formule (II), on peut citer, à titre d'exemples, l'isopentane ; le diméthyl-2,3 butane ; le méthyl-2 pentane ; le méthyl-2 hexane ; le méthylcyclopentane ; le méthylcyclohexane ; le diméthyl-1,4 cyclohexane ; la décaline ; et l'adamantane.

Dans le procédé suivant l'invention, il est nécessaire d'utiliser un formiate qui conduise, dans le milieu réactionnel, à un cation R²R³R⁴C⁺ stable. Ce cation stable est obtenu notamment dans le cas où le carbone portant les radicaux R², R³ et R⁴ est un atome de carbone tertiaire (par exemple, si l'on utilise le formiate de tertiobutyle), ou bien dans le cas où cet atome de carbone est un atome de carbone secondaire et où le cation résultant se réarrange en cation tertiaire (par exemple, si l'on utilise le formiate d'isobutyle, où le cation isobutyle se réarrange en cation tertiobutyle).

Une condition supplémentaire est cependant que le composé R²R³R⁴CH puisse facilement s'éliminer du milieu, ce qui déplace l'équilibre dans le sens de la formation du composé (I). De ce fait, les cations pour lesquels R², R³ et R⁴ représentent un groupe aryle ne conviennent pas, de même que ceux dans lesquels R², R³ et R⁴ représentent chacun un reste alkyle totalisant par exemple plus de 6 atomes de carbone.

Par «alkyle» dans la définition de la formule (III), on entend ici généralement les groupes alkyle en C₁ à C₆, en particulier les groupes, alkyle inférieurs en C₁-C₆ et les groupes cycloalkyle en C₅, C₆.

Ainsi, comme formiate de formule (III), on peut utiliser notamment le formiate de tertiobutyle ou le formiate d'isobutyle, car tous deux donnent de l'isobutane, lequel est gazeux dans tout le domaine de températures utilisé et se dégage facilement du milieu réactionnel, déplaçant ainsi l'équilibre vers la formation de l'acide carboxylique.

Pour catalyser la réaction, on utilise, de préférence, au moins un acide minéral, qui peut être notamment l'acide sulfurique ou l'acide phosphorique, ou bien un mélange des deux, cet acide étant mis en oeuvre à l'état liquide, avec une concentration ou force de préférence au moins égale à 80% et allant jusqu'à 100%. On préfère l'acide sulfurique à une force d'au moins 95%.

Le procédé de préparation d'un acide carboxylique selon l'invention consiste à ajouter le formiate (III) à un mélange de l'alcane (II) et d'au moins un acide comme catalyseur, et à agiter énergiquement le milieu réactionnel à la température ambiante ou légèrement supérieure à l'ambiante. Une agitation énergique est généralement souhaitable pour la bonne marche de la réaction, en raison de l'existence de deux phases. Le rapport molaire alcane (II)-formiate (III) se situe généralement entre environ 0,5 et 3. On préfère toutefois travailler avec un excès d'alcane, ce qui améliore le rendement. La température de réaction n'est pas un paramètre critique ; elle peut être comprise entre environ 0°C et la température de reflux de l'alcane à carbonyler, par exemple entre environ 0°C et environ 80°C ; on peut notamment effectuer cette réaction à reflux du solvant, par exemple à une température d'environ 65-70°C dans le cas où l'on utilise CCl₄ comme solvant. Sinon, il n'y a aucun inconvénient à conduire la réaction à température ambiante.

La durée de réaction ne constitue pas non plus un paramètre critique : on peut mentionner, en règle générale, des temps de réaction allant de 1 heure à 5 heures.

Le procédé selon la présente invention peut être conduit en milieu solvant. Un solvant du type tétrachlorure de carbone est utilisé habituellement, mais il n'est pas indispensable, pouvant être remplacé par un excès d'alcane. Ce résultat confirme que le tétrachlorure de carbone ne joue pas ici un rôle chimique de promoteur.

Le procédé de la présente invention peut être conduit en présence d'au moins un catalyseur supplémentaire de type oxyde ou sulfate métallique comme, par exemple, Ag₂O, Cu₂O, FeO ou FeSO₄.

On conduit généralement la réaction selon l'invention à pression atmosphérique ; on peut cependant effectuer cette réaction sous une pression pouvant atteindre jusqu'à 60 bars environ, par exemple sous pression d'azote ou d'oxyde de carbone supplémentaire, bien que dans ces conditions le dégagement de l'alcane R₂R₃R₄CH soit ralenti, ce qui peut avoir un effet préjudiciable sur le rendement de la réaction.

A titre indicatif, on met en contact environ 20 à 150 ml d'acide sulfurique concentré pour 0,1 à 0,5 mole d'alcane à carbonyler ; on ajoute de 0,05 à 0,5 mole de formiate, et on agite vigoureusement le milieu réactionnel à une température comprise de préférence entre 10°C et 40°C environ.

Le traitement et la purification du produit réactionnel brut s'effectuent facilement par les méthodes habituelles d'extraction des acides carboxyliques. On peut ainsi diluer le produit réactionnel brut avec de l'eau glacée, décanter et extraire la phase aqueuse par exemple au moyen de tétrachlorure de carbone. Les phases organiques réunies sont alors extraites à l'aide d'une base forte telle qu'un hydroxyde alcalin (soude, potasse) pour former le sel alcalin d'acide carboxylique qui passe en phase aqueuse. L'acide est décanté par traitement à l'aide d'un acide fort tel que l'acide chlorhydrique, puis extraction de la phase aqueuse par traitement par exemple, par le chloroforme. Le solvant est évaporé du milieu qui contient l'acide carboxylique attendu.

Les exemples suivants illustrent l'invention sans toutefois la limiter. Dans ces exemples, les pourcentages sont donnés en poids.

### Exemple 1

Dans un réacteur de 250 ml, contenant 100 ml d'acide sulfurique concentré et 0,3 mole de méthylcyclohexane, on ajoute 0,2 mole de formiate d'isobutyle ; le mélange réactionnel est agité vigoureusement à 26°C, pendant 4 heures. Ensuite, le mélange est versé dans 200 g de glace pilée ; on extrait la phase aqueuse avec deux fois 50 ml de tétrachlorure de carbone. Les phases organiques sont traitées par de la soude 2N jusqu'à pH=9. La phase aqueuse basique est réacidifiée avec de l'acide chlorhydrique 1N jusqu'à pH=1. On extrait les acides avec deux fois 100 ml de chloroforme. Les phases organiques obtenues sont réunies et traitées à l'évaporateur rotatif pour éliminer le solvant. Le rendement en acide méthyl-1 cyclohexylcarboxylique est de 34,8%.

### Exemple 2

On procède comme à l'Exemple 1, à ceci près que l'on introduit 50 ml d'acide sulfurique et que l'on rajoute 0,1 mole d'acide formique. Le rendement en acide méthyl-1 cyclohexylcarboxylique est de 37,5%.

### Exemple 3

On procède comme à l'Exemple 1, à ceci près que l'on introduit 50 ml d'acide sulfurique et que la réaction est effectuée à 20°C. Le rendement en acide méthyl-1 cyclohexylcarboxylique est de 45,2%.

### Exemple 4

On procède comme à l'Exemple 1, à ceci près que l'on introduit 50 ml d'acide sulfurique, que l'on rajoute 0,1 mole d'acide formique et 0,1 g d'oxyde de cuivre, et que la réaction est effectuée à 20°C. Le rendement en acide méthyl-1 cyclohexylcarboxylique est de 67,7%.

### Exemple 5

On procède comme à l'Exemple 1, à ceci près que l'on introduit 50 ml d'acide sulfurique et que la réaction est effectuée à 30°C. Le rendement en acide méthyl-1 cyclohexylcarboxylique est de 57,5%.

### Exemple 6

On procède comme à l'Exemple 1, à ceci près que l'on introduit 50 ml d'acide sulfurique et que la réaction est effectuée à 40°C. Le rendement en acide méthyl-1 cyclohexylcarboxylique est de 40%.

### Exemple 7

On procède comme à l'Exemple 1, à ceci près que l'on introduit 50 ml d'acide sulfurique et que la réaction est effectuée à 10°C. Le rendement en acide méthyl-1 cyclohexylcarboxylique est de 14%.

### Exemple 8

On procède comme à l'Exemple 1, à ceci près que l'on introduit 50 ml d'acide sulfurique et que la réaction est effectuée à 30°C, en présence de 0,2 mole d'acide formique pour 0,1 mole de formiate d'isobutyle. Le rendement en acide méthyl-1 cyclohexylcarboxylique est de 61%.

### Exemple 9

On procède comme à l'Exemple 1, à ceci près que l'on introduit 50 ml d'acide sulfurique et que la réaction est effectuée à 30°C, en présence de 0,1 g d'oxyde de cuivre. Le rendement en acide méthyl-1 cyclohexylcarboxylique est de 45%.

### Exemple 10

On procède comme à l'Exemple 1, à ceci près que l'on introduit 32 ml d'acide sulfurique et que la réaction est effectuée à 30°C. Le rendement en acide méthyl-1 cyclohexylcarboxylique est de 23%.

### Exemple 11

On procède comme à l'Exemple 3, à ceci près que l'alcane introduit est le diméthyl-2,3-butane. Le rendement en acide triméthyl-2,2,3 butanoïque est de 43,3%.

### Exemple 12

On procède comme à l'Exemple 11, à ceci près que la réaction est conduite à 30°C. Le rendement en acide triméthyl-2,2,3 butanoïque est de 30,7%.

### Exemple 13

On procède comme à l'Exemple 5, à ceci près que l'alcane introduit est la décaline. Le rendement en acide décalylcarboxylique est de 40%.

### Exemple 14

On procède comme à l'Exemple 5, à ceci près que l'on utilise du formiate de tertiobutyl à une température de 15°C. Le rendement en acide méthyl-1 cyclohexylcarboxylique est de 30%.

### Exemple 15

On procède comme à l'Exemple 5, à ceci près que l'on introduit un mélange de 20 ml d'acide phosphorique et de 50 ml d'acide sulfurique ; la température de réaction est de 30°C. Le rendement en acide méthyl-1 cyclohexylcarboxylique est de 30%.

### Exemple 16

On procède comme à l'Exemple 5, à ceci près que l'on utilise un rapport molaire méthylcyclohexane - formiate d'isobutyle égal à 2. Le rendement en acide méthyl-1 cyclohexylcarboxylique est de 41%.

### Exemple 17

On procède comme à l'Exemple 5, à ceci près que l'on utilise un rapport molaire méthylcyclohexane - formiate d'isobutyle égal à 1. Le rendement en acide méthyl-1 cyclohexylcarboxylique est de 43,8%.

### Exemple 18

On procède comme à l'Exemple 5, à ceci près que l'on introduit 0,6 mole d'eau. Le rendement en acide méthyl-1 cyclohexylcarboxylique est de 10%.

### Exemple 19

On procède comme à l'Exemple 1, à ceci près que l'on travaille sous une pression de 26 bars d'oxyde de carbone. Le rendement en acide méthyl-1 cyclohexylcarboxylique est de 16%.

### Exemple 20

On procède comme à l'Exemple 5, à ceci près que l'alcane introduit est l'adamantane. Le rendement en acide adamantylcarboxylique est de 90%.

### Exemple 21

On procède comme à l'Exemple 5, à ceci près que l'alcane introduit est l'adamantane et que l'on introduit 0,15 mole d'eau. Le rendement en acide adamantylcarboxylique est de 80%.

## Revendications

1. Procédé de fabrication d'un acide carboxylique représenté par la formule (I) ci-après :
R¹ - COOH (I)
dans laquelle R¹ représente un reste alkyle ramifié tertiaire ou bien un reste cycloalkyle à un ou plusieurs cycles accolés ou non, et éventuellement substitué par au moins un reste alkyle, l'atome de carbone en alpha du groupe carbonyle étant un atome de carbone tertiaire,
caractérisé par le fait que l'on fait réagir, en présence d'un acide comme catalyseur, un alcane ramifié ou cyclique de formule (II) :
R¹H (II)
dans laquelle R¹ est un reste alkyle ramifié ou bien un reste cycloalkyle à un ou plusieurs cycles accolés ou non, et éventuellement substitué par au moins un reste alkyle, l'atome de carbone en alpha de l'atome d'hydrogène étant un atome de carbone tertiaire ou un atome de carbone secondaire capable de se réarranger en cours de réaction en atome tertiaire,
avec un formiate de formule (III) : 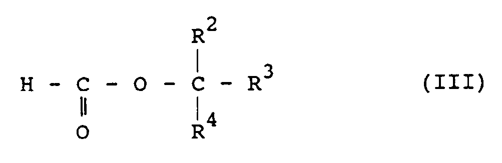 dans laquelle R², R³ et R⁴ représentent chacun indépendamment un atome d'hydrogène ou un reste alkyle, à la triple condition que R², R³ et R⁴ ne représentent pas simultanément un atome d'hydrogène, que le composé (III) conduise dans le milieu réactionnel, directement, ou à la suite d'un réarrangement, à un cation R²R³R⁴C⁺ stable, et que l'alcane R²R³R⁴CH qui se forme puisse s'éliminer aisément du milieu réactionnel.

2. Procédé selon la revendication 1, caractérisé par le fait qu'on utilise, comme alcane (II), un alcane de formule (IIa) : 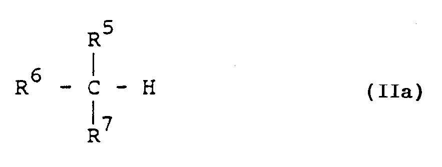 dans laquelle :
- R⁵, R⁶ et R⁷ représentent chacun indépendamment un reste alkyle en C₁-C₁₀ ; ou bien
- deux radicaux des R⁵ à R⁷ sont réunis pour former un enchaînement -(CH₂)ₙ- , n étant un nombre entier allant de 4 à 11, et le troisième représente un reste alkyle en C₁-C₇ ; ou bien
- R⁵, R⁶ et R⁷ sont combinés entre eux et avec l'atome de carbone qui les porte pour former un reste cycloalkyle à un, deux ou trois cycles.

3. Procédé selon la revendication 2, caractérisé par le fait qu'on utilise, comme alcane de formule (II), l'isopentane ; le diméthyl-2,3 butane ; le méthyl-2 pentane; le méthyl-2 hexane ; le méthylcyclopentane; le méthylcyclohexane ; le diméthyl-1,4 cyclohexane ; la décaline ; ou l'adamantane.

4. Procédé selon l'une des revendications 1 à 3, caractérisé par le fait qu'on utilise, comme formiate de formule (III), le formiate de tertiobutyle ou le formiate d'isobutyle.

5. Procédé selon l'une des revendications 1 à 4, caractérisé par le fait qu'on utilise, comme catalyseur, au moins un acide minéral liquide ayant une concentration au moins égale à 80%.

6. Procédé selon la revendication 5, caractérisé par le fait qu'on utilise, comme catalyseur, un acide sulfurique ayant une concentration au moins égale à 95%.

7. Procédé selon l'une des revendications 1 à 6, caractérisé par le fait qu'on conduit la réaction avec un rapport molaire alcane (II)/formiate (III) compris entre 0,5 et 3 .

8. Procédé selon l'une des revendications 1 à 7, caractérisé par le fait qu'on conduit la réaction en milieu solvant.

9. Procédé selon l'une des revendications 1 à 8, caractérisé par le fait qu'on travaille à une température comprise entre 0°C et la température de reflux de l'alcane (II) à carbonyler, ou encore à la température de reflux du solvant, lorsqu'un solvant est utilisé.

10. Procédé selon l'une des revendications 1 à 9, caractérisé par le fait qu'on conduit la réaction pendant une durée allant de 1 heure à 5 heures.

11. Procédé selon l'une des revendications 1 à 10, caractérisé par le fait qu'on conduit la réaction en présence d'au moins un catalyseur supplémentaire de type oxyde ou sulfate métallique.

## Patentansprüche

1. Verfahren zur Herstellung einer Carbonsäure nach der nachstehenden Formel (I):
R¹-COOH (I)
in der R¹ einen verzweigten tertiären Alkylrest oder einen Cycloalkylrest mit einem oder mehreren Ringen, die aneinanderhängen oder nicht, und gegebenenfalls durch mindestens einen Alkylrest substituiert, darstellt und das Kohlenstoffatom in der α-Stellung der Carbonylgruppe ein tertiäres Kohlenstoffatom ist,
dadurch gekennzeichnet, daß man in Gegenwart einer Säure als Katalysator ein verzweigtes oder zyklisches Alkan der Formel (II):
R¹H (II)
in der R¹ ein verzweigter Alkylrest oder auch ein Cycloalkylrest mit einem oder mehreren Ringen, die aneinanderhängen oder nicht, und gegebenenfalls durch mindestens einen Alkylrest substituiert ist und das Kohlenstoffatom in der α-Stellung des Wasserstoffatoms ein tertiäres Kohlenstoffatom oder ein sekundäres Kohlenstoffatom ist, das imstande ist, sich im Zuge der Reaktion zu einem tertiären Atom umzulagern,
zur Reaktion bringt mit einem Formiat der Formel (III) 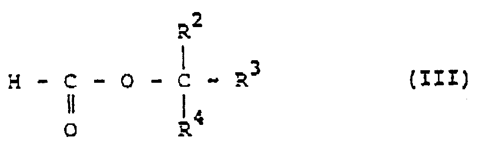 in der R², R³ und R⁴ jeweils unabhängig ein Wasserstoffatom oder einen Alkylrest darstellen, unter den drei Voraussetzungen, daß R², R³ und R⁴ nicht gleichzeitig ein Wasserstoffatom darstellen, daß die Verbindung (III) in dem Reaktionsmilieu unmittelbar oder infolge einer Umlagerung zu einem stabilen R²R³R⁴C⁺-Kation führt, und daß das sich bildende Alkan R²R³R⁴CH sich leicht von dem Reaktionsmilieu trennen kann.

2. Verfahren nach Anspruch 1, dadurch gekennzeichnet, daß man als Alkan (II) ein Alkan der Formel (IIa) einsetzt: 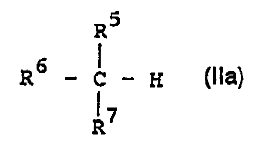 in der
- R⁵, R⁶ und R⁷ jeweils unabhängig einen C₁-C₁₀-Alkylrest darstellen; oder
- zwei der Reste R⁵ bis R⁷ vereinigt sind, um eine Kette -(CH₂)ₙ- zu bilden, wobei n eine ganze Zahl im Bereich von 4 bis 11 ist, und der dritte Rest ein C₁-C₇-Rest ist; oder
- R⁵, R⁶ und R⁷ miteinander und mit dem sie tragenden Kohlenstoffatom kombiniert sind, um einen Cycloalkylrest mit einem, zwei oder drei Ringen zu bilden.

3. Verfahren nach Anspruch 2, dadurch gekennzeichnet, daß man als Alkan der Formel (II) Isopentan, Dimethyl-2,3 butan, Methyl-2 pentan, Methyl-2 hexan, Methylcyclopentan, Methylcyclohexan, Dimethyl-1,4 cyclohexan, Decalin oder Adamantan verwendet.

4. Verfahren nach einem der Ansprüche 1 bis 3, dadurch gekennzeichnet, daß man als Formiat der Formel (III) tert.-Butylformiat oder Isobutylformiat verwendet.

5. Verfahren nach einem der Ansprüche 1 bis 4, dadurch gekennzeichnet, daß man als Katalysator mindestens eine flüssige Mineralsäure mit einen Konzentration von mindestens 80% verwendet.

6. Verfahren nach Anspruch 5, dadurch gekennzeichnet, daß man als Katalysator eine Schwefelsäure mit einer Konzentration von mindestens 95% verwendet.

7. Verfahren nach einem der Ansprüche 1 bis 6, dadurch gekennzeichnet, daß man die Reaktion mit einem Molverhältnis Alkan (II)/Formiat (III) von 0,5 bis 3 durchführt.

8. Verfahren nach einem der Ansprüche 1 bis 7, dadurch gekennzeichnet, daß man die Reaktion in einem Lösungsmittelmilieu durchführt.

9. Verfahren nach einem der Ansprüche 1 bis 8, dadurch gekennzeichnet, daß man bei einer Temperatur zwischen 0°C und der Rückflußtemperatur des zu carbonylisierenden Alkans (II) oder auch, sofern ein Lösungsmittel verwendet wird, bei Rückflußtemperatur des Lösungsmittels arbeitet.

10. Verfahren nach einem der Ansprüche 1 bis 9, dadurch gekennzeichnet, daß man die Reaktion für ein Dauer von 1 bis 5 Stunden durchführt.

11. Verfahren nach einen der Ansprüche 1 bis 10, dadurch gekennzeichnet, daß man die Reaktion in Gegenwart eines zusätzlichen Katalysators des Metalloxid- oder Metallsulfattyps durchführt.

## Claims

1. Process for the manufacture of a carboxylic acid represented by the formula (I) below:
R¹ - COOH (I)
in which R¹ represents a branched tertiary alkyl residue or else a cycloalkyl residue containing one or a number of rings which may or may not be fused, and optionally substituted by at least one alkyl residue, the carbon atom α to the carbonyl group being a tertiary carbon atom,
characterised in that a branched or cyclic alkane of formula (II):
R¹H (II)
in which R¹ is a branched alkyl residue or else a cycloalkyl residue containing one or a number of rings which may or may not be fused, and optionally substituted by at least one alkyl residue, the carbon atom a to the hydrogen atom being a tertiary carbon atom or a secondary carbon atom capable of rearranging to a tertiary atom during reaction,
is reacted, in the presence of an acid as catalyst, with a formate of formula (III): 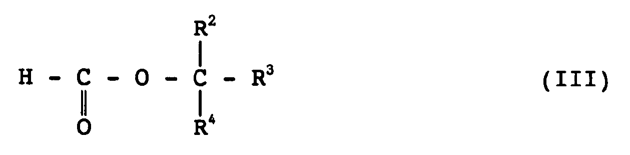 in which R², R³ and R⁴ each independently represent a hydrogen atom or an alkyl residue, with the three- fold condition that R², R³ and R⁴ do not all represent a hydrogen atom, that the compound (III) leads in the reaction mixture, directly or following a rearrangement, to a stable cation R²R³R⁴C⁺, and that the alkane R²R³R⁴CH which is formed can be easily removed from the reaction mixture.

2. Process according to Claim 1, characterised in that there is used, as alkane (II), an alkane of formula (IIa): 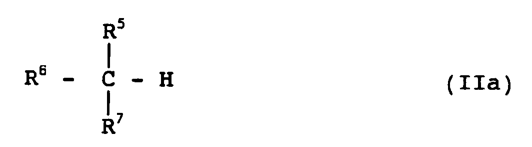 in which:
- R⁵, R⁶ and R⁷ each independently represent a C₁-C₁₀ alkyl residue; or else
- two radicals from R⁵ to R⁷ are combined to form a linkage -(CH₂)ₙ-, n being an integer ranging from 4 to 11, and the third represents a C₁-C₇ alkyl residue; or else
- R⁵, R⁶ and R⁷ are combined with each other and with the carbon atom which carries them to form a cycloalkyl residue containing one, two or three rings.

3. Process according to Claim 2, characterised in that there is used, as alkane of formula (II), isopentane, 2,3-dimethylbutane, 2-methylpentane, 2-methylhexane, methylcyclopentane, methylcyclohexane, 1,4-dimethylcyclohexane, decalin or adamantane.

4. Process according to one of Claims 1 to 3, characterised in that there is used, as formate of formula (III), tert-butyl formate or isobutyl formate.

5. Process according to one of Claims 1 to 4, characterised in that there is used, as catalyst, at least one liquid inorganic acid having a concentration at least equal to 80 %.

6. Process according to Claim 5, characterised in that there is used, as catalyst, a sulphuric acid having a concentration at least equal to 95 %.

7. Process according to one of Claims 1 to 6, characterised in that the reaction is carried out with an alkane (II)/formate (III) molar ratio of between 0.5 and 3.

8. Process according to one of Claims 1 to 7, characterised in that the reaction is carried out in solvent medium.

9. Process according to one of Claims 1 to 8, characterised in that the processing is carried out at a temperature of between 0°C and the reflux temperature of the alkane (II) to be carbonylated, or even at the reflux temperature of the solvent, when a solvent is used.

10. Process according to one of Claims 1 to 9, characterised in that the reaction is carried out for a period of time ranging from 1 hour to 5 hours.

11. Process according to one of Claims 1 to 10, characterised in that the reaction is carried out in the presence of at least one additional catalyst of metal oxide or sulphate type.
